Europäisches Patentamt

European Patent Office

Office européen des brevets

(11) Publication number: **0 189 990**
A2

# EUROPEAN PATENT APPLICATION

(21) Application number: 86300283.8

(22) Date of filing: 16.01.86

(51) Int. Cl.⁴: **C 07 D 475/04**

(30) Priority: 29.01.85 US 695949

(43) Date of publication of application:
06.08.86 Bulletin 86/32

(84) Designated Contracting States:
CH DE FR GB IT LI

(71) Applicant: DANA-FARBER CANCER INSTITUTE
44 Binney Street
Boston Massachusetts 02115(US)

(72) Inventor: Rosowsky, Andre
76 Lindbergh Avenue
Needham Massachusetts(US)

(72) Inventor: Forsch, Ronald A.
335 Huntington Avenue
Boston Massachusetts(US)

(74) Representative: Bizley, Richard Edward et al,
BOULT, WADE & TENNANT 27 Furnival Street
London EC4A 1PQ(GB)

(54) Single stage reduction and formylation of folic or homofolic acid.

(57) Folic or homofolic acid is converted to the corresponding
$N^5$-formyl-5,6,7,8-tetrahydrofolic or-homofolic acid by reaction in solution in excess formic acid at 0°–15°C with a secondary amine complex of borane.

EP 0 189 990 A2

## SINGLE STAGE REDUCTION AND FORMYLATION OF FOLIC OR

## HOMOFOLIC ACID

This invention relates to a one-step procedure for conversion of folic or homofolic acid to the corresponding $N^s$-formyl-5,6,7,8-tetrahydro acid and pertains more specifically to concomitant formylation and reduction of folic or homofolic acid, using a sec. amine-borane complex, in excess formic acid as the reaction medium.

It has previously been proposed to formylate and reduce folic acid in excess formic acid using platinum oxide as catalyst, in a two-stage procedure in which the first stage involves hydrogenation of folic acid in excess formic acid using platinum oxide catalyst and the second stage involves treating the reaction mixture with strong alkali, as described in Shive U.S. Patent 2,741,608, D'Amato U.S. Patent 2,927,113, and Roth et al., J.Am. Chem. Soc., Vol. 74, 3247-3252 (1952). A two-stage procedure has also been proposed in which the first stage involves reduction of folic acid with sodium borohydride, followed by treatment of the tetrahydrofolic acid with formic acid, as described in Temple et al., U.S. Patent 4,148,999. Another two-stage procedure as described by Moran et al., Anal.Biochem., Vol. 122, 70-78 (1982) provides enzymatic reduction of folic acid followed by reaction with formic acid in the presence of a carbodiimide.

It has also been proposed in Martinelli et al., Preparat. Biochem., Vol. 10, 161-166 (1980) to convert folic acid to tetrahydrofolic acid by heating it with dimethylamine-borane complex in glacial acetic acid; and by Moran et al., AACR Abstracts 1984, Preclin. Pharmacol. and Exp. Therapeut., p.311 (March, 1984) to reduce folic acid to tetrahydrofolic acid with dimethylamine-borane complex or with enzyme followed by formylation by a carbodiimide-mediated reaction. In addition, the use of boron triformate as a formylating agent has been proposed by Gertsev, Chem.Abstr., Vol. 73, 87373c (1970); Vol. 97, 162750z (1982).

It has now been found that folic acid and homofolic acid are converted to the corresponding $N^5$-formyl-5,6,7,8-tetrahydro acids in a single stage when treated in solution in an excess of formic acid as the reaction medium with a complex of borane and a secondary amine at a temperature of 0°-15°C, preferably 0°-5°C. By "excess" is meant an amount in excess of the stoichiometric quantity required for the formylation reaction.

Any of the various well known borane complexes with secondary amines may be used in the present invention, such as dimethylamine borane, diisopropylamine borane, pyrrolidine borane, morpholine borane, poly(2-vinylpyridine) borane, or the like, of which dimethylamine borane is preferred.

While it is not essential that anhydrous formic acid be used as the reaction medium, the fact that water is one of the products of the formylation reaction makes it desirable to avoid the presence of excessive water in order to drive the formylation reaction to completion. In general, the less water present in the reaction medium at the outset, the better, and for this reason it is preferred to employ commercially available 97%-100% formic acid as the medium.

The temperature of the reaction mixture must be kept low, from 0° to 15°C, preferably from 0° to 5°C, in order to minimize or prevent cyclodehydration and the formation of $N^5$, $N^{10}$-methenyl-5,6,7,8-tetrahydro folic or homofolic acid.

The amount or extent of the excess of formic acid beyond that stoichiometrically required is not critical and may vary over a wide range, the minimum being amount sufficient to dissolve the folic or homofolic acid, and the maximum being limited only by economic considerations and/or the need easily to separate the desired product from the reaction mixture.

The amount of the secondary amine borane complex employed is also preferably in excess of the amount theoretically required for hydrogenation in order to ensure

-4-

completion of the hydrogenation, the maximum extent of the excess being limited by economic considerations. In general, an excess of 10% to 2000% above the theoretical amount is preferred.

The reaction is essentially complete in less than twenty four hours at 5°C.

The formylated and reduced products may be isolated from the reaction mixture by conventional procedures in the form of the free acids or in the form of the commercially desirable calcium salt or in the form of other divalent salts such as magnesium, barium, or zinc salts, as is well known in the art.

The following examples are intended to illustrate more fully the nature of the invention without acting as a limitation upon its scope.

Example 1

Leucovorin Calcium Salt. A solution of folic acid (477 mg, 1 mmol) in 97-100% formic acid (10 mL) was cooled in an ice bath and treated with $BH_3.HNMe_2$ (885 mg, 15 mmol) in small portions over 1 h with vigorous stirring. When addition was complete the light orange solution was left to stand in the refrigerator at 4°C for 24 h. The solution was then poured with stirring into ether (50 mL), the ether was decanted, and the residue was triturated with fresh ether until a powder was obtained. After decantation of the ether, water (10 mL) was added and calcium hydroxide was added to the suspension in small portions with stirring until the pH was strongly alkaline. Final solubilization was achieved with the aid of an ultrasonic bath. The pH was adjusted to 7.5 with dilute formic acid, a small amount of fine insoluble material was filtered off, and the filtrate was diluted with ethanol (120 mL). After several hours of refrigeration the crude product was collected, reprecipitated once more from aqueous ethanol, and dried in vacuo at 65°C over $P_2O_5$ to obtain a white solid (396 mg, 64% yield); $R_f$ 0.7 (cellulose, 3% $NH_4Cl$); IR (KBr) 3400, 1610-1640 cm$^{-1}$; NMR $\delta$ ($D_2O$) 1.5-3.4 (m, alkyl), 6.60 (d, J=8 Hz, aryl protons <u>ortho</u> to $N^{10}$), 7.57 (d, J=8 Hz, aryl protons <u>ortho</u> to CONH); UV: $\lambda_{max}$ ($H_2O$) 285 nm ($\epsilon$ 25,700). Anal. Calcd for $C_{20}H_{21}N_7O_7Ca.0.25$ $Ca(OOCH)_2.4.5H_2O$: C, 39.39; H, 4.92; N, 15.68; Ca, 8.01. Found: C, 39.52; H, 5.26; H, 15.78; Ca, 8.01.

Example 2

5-Formyl-5,6,7,8-tetrahydrohomofolic Acid, Calcium Salt. A solution of homofolic acid (491 mg, 1 mmol) in 10 mL 97-100% formic acid was cooled in an ice bath. Then $BH_3.HNMe_2$ (118 mg, 2 mmol) was added all at once with vigorous stirring at 15 min intervals for a total of eight times (16 mmol). When all the additions were complete, the solution was placed in the refrigerator (4°C) overnight before being poured into ether (100 mL) with stirring. The ether was decanted and the residue was washed repeatedly with ether and dissolved in water (5 mL). A minimum amount of $NH_4OH$ was added to dissolve the compound, and $CaCl_2.2H_2O$ (1 g) was added, followed by ethanol (100 mL). After overnight refrigeration the collected product was dissolved in water (15 mL) with the help of a sonicating bath. A small amount of insoluble material was removed by filtration, the filtrate was decolorized with charcoal, and the product was reprecipitated with ethanol (100 mL), collected, and dried in vacuo at 60-65°C over $P_2O_5$ to give a colorless powder (375 mg, 60% yield); $R_f$ 0.7 (cellulose, 3% $NH_4Cl$); IR (KBr) $\nu$ 3390, 1610-1640, 1560 cm$^{-1}$; NMR $\delta$ ($D_2O$) 1.5-2.7 and 2.9-3.7 (m, alkyl), 6.87 (d, J=8 Hz, aryl protons ortho to $N^{10}$), 7.87 (d, J=8 Hz, aryl protons ortho to CONH); UV $\lambda$ max ($H_2O$) 285 nm ($\epsilon$ 26,000). Anal. Calcd for $C_{21}H_{23}N_7O_7Ca.5.5H_2O$: C, 40.38; H, 5.49; N, 15.70; Ca, 6.42. Found: C, 40.38; H, 5.38; N, 15.45; Ca, 6.44.

CLAIMS:

1. A method of formylating and reducing folic or homofolic acid to form the corresponding $N^5$-formyl-5,6,7,8-tetrahydrofolic or tetrahydrohomofolic acid product which comprises reacting said folic or homofolic acid in solution in excess formic acid at a temperature of 0° to 15°C with a secondary amine complex of borane.

2. A method as claimed in claim 1 in which said complex is dimethyl amine borane.

3. A method as claimed in claim 1 or claim 2 in which said temperature is 0° to 5°C.

4. A method as claimed in claim 3 in which said product is isolated from the reaction mixture in the form of its calcium salt.

5. A method as claimed in any one of claims 1 to 4 in which the amount of said secondary amine complex of borane employed is from 10% to 2000% in excess of the amount theoretically required for hydrogenation.